# EUROPEAN PATENT SPECIFICATION

(11) **EP 3 005 970 B1**
(45) Date of publication and mention of the grant of the patent: **08.01.2020**
(21) Application number: 14800625.7
(22) Date of filing: 23.05.2014
(51) Int. Cl.: A61B 90/50

(54) **STAND HAVING COUNTERBALANCE PART**
STÄNDER MIT GEGENGEWICHTSELEMENT
SUPPORT AVEC ÉLÉMENT D'ÉQUILIBRAGE

(30) Priority: 24.05.2013 KR 20130059337; 22.05.2014 KR 20140061915
(43) Date of publication of application: 13.04.2016
(60) Divisional application: 17166173.9
(73) Proprietor: Koh Young Technology Inc., Seoul 153-706 (KR); Industry-University Cooperation Foundation Hanyang University ERICA Campus, Ansan-si, Gyeonggi-do 426-791 (KR)
(72) Inventor: YI, Byung-Ju, Bucheon-si Gyeonggi-do 420-709 (KR); SEO, Jong-Tae, Ansan-si Gyeonggi-do 426-896 (KR)
(74) Representative: Zardi, Marco
(86) International application number: PCT/KR2014/004649
(87) International publication number: WO 2014/189336

(56) References cited:
- EP-A1- 0 609 085
- DE-A1- 19 742 051
- DE-U1- 9 321 575
- JP-A- H1 128 216
- JP-A- H06 197 912
- JP-A- H06 217 993
- JP-A- H10 272 143
- US-A- 5 528 417
- US-A1- 2004 172 012
- US-A1- 2004 190 131
- US-A1- 2008 237 413

## Description

### TECHNICAL FIELD

The present invention relates to a stand equipped with a counterbalance unit, more particularly, a stand equipped with a counterbalance unit wherein a medical apparatus such as a microscope and a surgical end effector can be installed and moved to a desired position.

### BACKGROUND

Microsurgery, in which a medical surgical microscope is used to observe affected parts while performing surgery, has been studied and introduced in the surgical operation field.

In such microsurgery, a stand is needed to install weighty objects, i.e., a surgical microscope with its attached devices; place them in a desired space; then maintain their position.

Generally, in such a stand, the middle part of a link unit using a parallel link is rotatably connected to a holding unit, while a surgical microscope is installed at one end of the link unit and has a balanced structure having a counterweight which is installed at the other one end of the surgical microscope to counterbalance a weight of the surgical microscope around a rotation point.

Since accessories, such as an assistant scope or a video camera, etc., are installed in the surgical microscope, an overall balance adjustment operation is carried out by changing a position of the counterweight according to its weight such that the surgical microscope and the counterweight are balanced.

However, in case when the surgical microscope and its attached devices remain in the desired position, their vertical balance needs to be maintained. However, a conventional stand has difficulties in controlling the vertical balance since the total weight of the surgical microscope is inconsistent due to the presence and absence of various attachments.

DE 197 42 051 A1 discloses Stand for movable appliance wherein a second stand part is movable in relation to a first stand part, and a swivel arm, attached to the first stand part, swivels on a swivel axle, and an energy store in the form of a compression spring exerts force on a fixed point on the swivel arm. EP0609085 discloses a stand for a medical device with a parallelogram linkage.

### SUMMARY

The present invention is devised to solve the problem stated above. The object of the present invention is to provide a stand equipped with a counterbalance unit capable of maintaining a reliable and efficient counterbalance regardless of positions of a medical apparatus.

Another object of the present invention is to provide a stand equipped with a replaceable counterweight based on the amount of a torque in joints caused by a medical apparatus, and a counterbalance unit capable of adjusting the length of a balance link.

A further object of the present invention is to provide a stand equipped with a counterbalance unit capable of adding degree of freedom easily.

In order to achieve the objects herein, a stand equipped with a counterbalance unit according to the present invention includes the features as claimed in appended independent claim 1. Preferred embodiments are described in the dependent claims.

The stand equipped with a counterbalance unit according to the present invention can obtain a large torque compensation effect with small weight by optimizing the mounting position of counterbalance.

In addition, even when a number of medical apparatus such as a microscope and the like are used, because of a replaceable counterweight based on the amount of the torque in joints caused by the medical apparatus and a counterbalance unit capable of adjusting the length of a balance link, a reliable and efficient counterbalance can be maintained.

Moreover, multiple extension link members can be installed according to the purpose of a user, thus increase the degree of freedom of the movement of the medical apparatus.

### BRIEF DESCRIPTION OF THE DRAWINGS

**FIG. 1** is a schematic diagram illustrating a stand equipped with a counterbalance unit according to the present invention.
**FIG. 2** is a schematic diagram illustrating **FIG. 1** in operation modes.
**FIG. 3** is a schematic diagram illustrating an embodiment of a counterbalance unit.
**FIG. 4** is a schematic diagram illustrating a stand equipped with extension link members.
**FIG. 5** is a schematic diagram illustrating another embodiment of **FIG. 4****.**
**FIG. 6** is a schematic diagram illustrating **FIG. 5** in operation modes.
**FIGS. 7** to **9** are schematic diagrams illustrating various embodiments of a counterbalance unit.

### DETAILED DESCRIPTION

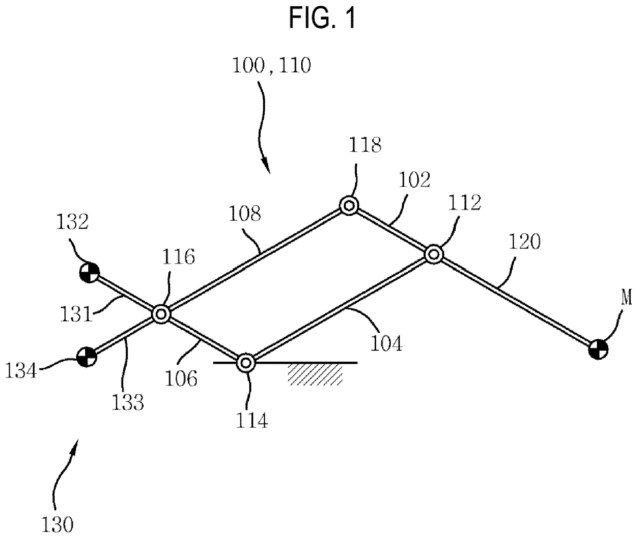

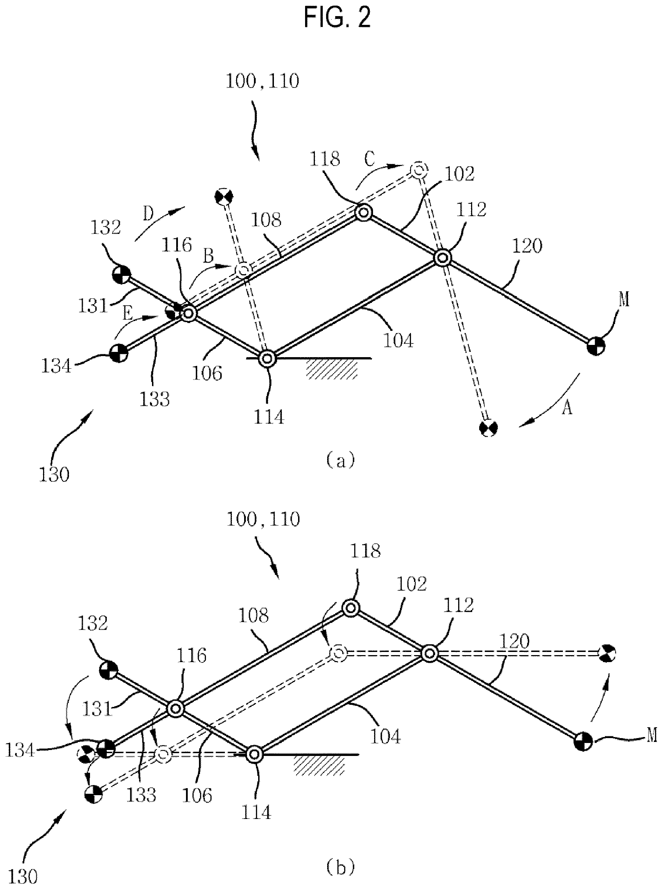

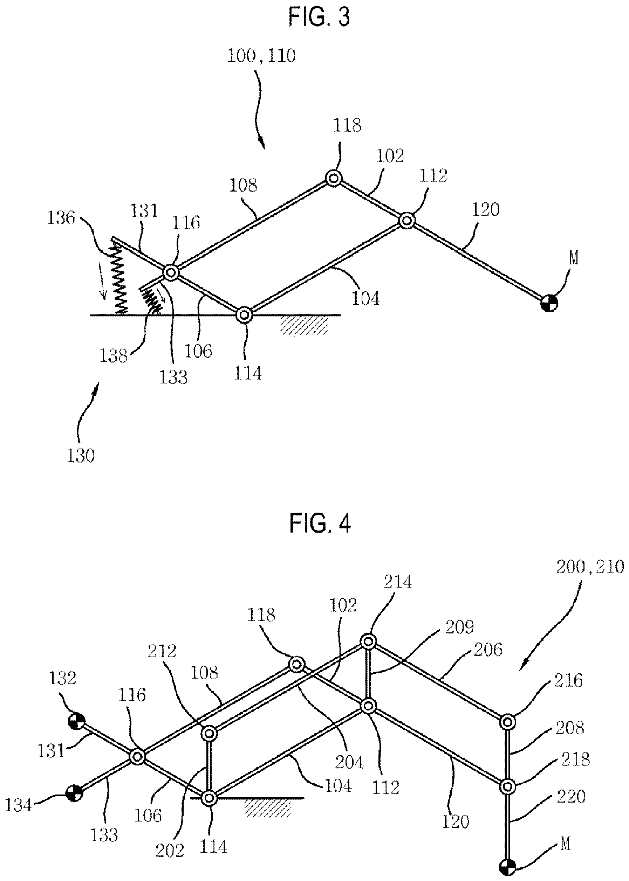

Hereinafter, more detailed description of a stand equipped with a counterbalance unit according to the present invention is provided with reference to appended drawings. The present invention relates to a stand equipped with a counterbalance unit. **FIG. 1** is a schematic diagram illustrating a stand equipped with a counterbalance unit not according to the present invention. **FIG. 2** is schematic diagrams illustrating **FIG. 1** in operation modes. **FIG. 3** is a schematic diagram illustrating an embodiment of a counterbalance unit.

A stand equipped with a counterbalance unit according to the present invention comprises: four links 100 arranged in a square configuration; four joints 110 that are connected to each connection part of the four links 100 respectively and enable these links 100 to be mutually rotatable; a front link 120 that is extended from an end of any one of the four links 100 with a medical apparatus M being mounted to a tip of the front link; a counterbalance unit 130 connected to a joint 110 that is diagonally located from the joint 110 from which the front link 120 is extended.

Each component is described in more details as follows.

Joints 110 comprise first, second, third and fourth joints 112, 114, 116, 118 and are connected to each connection part of the four links 100 respectively so that the links 100 are mutually rotatable.

The links 100 comprise a first link 102 whose both ends are connected to the first and fourth joints 112, 118; a third link 106 whose both ends are connected to the second and third joints 114, 116 and placed on the opposite side of the first link 102; a second link 104 whose both ends are connected to the first and second joints 112, 114; and a fourth link 108 whose both ends are connected to the third and fourth joints 116, 118 and placed on the opposite side of the second link 104. The links are arranged in a square configuration, but it is preferable that the links are arranged in a parallelogram configuration as illustrated in **FIG. 1****.** For simplicity, the description hereinafter relates to an embodiment in which the links 100 are arranged in a parallelogram configuration. In case where the links 100 are not formed in a parallelogram configuration, parallel can be understood as opposite.

Both ends of the first link 102 are connected to the first and fourth joints 112, 118, while the third link 106 is located in parallel to the first link 102 and both ends of the third link 106 are connected to the second and third joints 114, 116. In addition, both ends of the second link 104 are connected to the first and second joints 112, 114, and both ends of the fourth link 108 are connected to the second and third joints 114, 116. The second link 104 and the fourth link 108 are parallel with each other. Accordingly, the first, second, third and fourth links 102, 104, 106, 108 are mutually rotatable and thus a medical apparatus M which will be described below has a degree of freedom.

Meanwhile, at least any one of the four joints 110 is fixed to and supported by a holding unit (not shown)., and in case of an embodiment of the present invention, the second joint 114 is fixed to and supported by the holding unit.

A front link 120 is extended from an end of any one link of the four links 100 and the medical apparatus M is mounted at a tip of the front link. The front link is interlocked to the movement of the links 100 to move the medical apparatus M. In the embodiment of the present invention, the front link 120 is extended from an end of the first link 102 as illustrated in **FIG. 1****,** and the first joint 112 is connected between the first link 102 and the front link 120.

The counterbalance unit 130 is connected to the joint 110 which is placed in a diagonal direction from the joint 110 that is located at the extension part from which the front link 120 is extended. The counterbalance unit functions to counterbalance the medical apparatus M. That is, according to the embodiment of the links 100, as illustrated in **FIG. 1****,** the first joint 112 is placed at the extension part from which the front link 120 is extended, and the third joint 116 is positioned in the diagonal direction from the first joint 112, thus the counterbalance unit 130 is connected to the third joint 116.

The counterbalance unit 130 in the present invention can use weighters and springs to balance against the front link 120 with the medical apparatus M. Hereinafter, the description of the case using the weighters will be made first.

The counterbalance unit 130 comprises first and second balancing links 131, 133 extended from the two links 100, i.e., the third and fourth links 106, 108 connected to the third joint 116; and first and second counterweights 132, 134 respectively mounted at the tip of the first and second balancing links 131, 133. Accordingly, the first and second counterweights 132, 134 are placed on the opposite side of the medical apparatus M, centering on the second joint 114, to maintain balance. When the medical apparatus M moves in the direction opposite to gravity, the first and second counterweights 132, 134 move in the direction of gravitational force, thus compensating the torque in joints caused by the medical apparatus. Since the counterbalance unit 130 is connected to the third joint 116 as illustrated in **FIG. 1** instead of to the second joint 114 that is functioning as the central axis, the effective distance to the central axis (the distance between the central axis and the gravity vector acting on the counterweights) is maximized in most movements of the links 100, and thus the torque in joints generated by the medical apparatus can be compensated in full.

Meanwhile, the length of the first and second balancing links 131, 133 is adjustable, and each of the first and second counterweights 132, 134 is removably installed on the first and second balancing links 131, 133 respectively, thus the compensated torque in joints can be controlled in accordance with the length of the front link 120 or the weight of a medical apparatus M.

The counterbalance unit 130 in another embodiment comprises the first and second balancing links 131, 133 extended from the third and fourth links 106, 108; and first and second counter springs 136, 138 attached to the tip of the first and second balancing links 131, 133 respectively. For reference, the holding unit fixing the second joint 114 is extended in parallel with the ground. And, as illustrated in **FIG. 3****,** one end of the first counter spring 136 is connected to the tip of the first balancing link 131, while the other end of the first counter spring 136 is connected to the holding unit. In this case, the first counter spring 136 is a tension spring, and moves the first balancing link 131 in the gravitational direction, i.e. the direction of the arrow, thus compensating the torque in joints generated by the medical apparatus M. One end of the second counter spring 138 is connected to the tip of the second balancing link 133, while the other end of the second counter spring 138 is connected to the holding unit. The second counter spring 138 is also a tension spring and moves the second balancing link 133 in the direction of the arrow, thus compensating the torque in joints generated by the medical apparatus.

Also, when necessary, one end of the counter spring is connected to the third link 106 and the other end of the counter spring is connected to the holding unit.

Hereinafter, the operation mode of the stand equipped with a counterbalance unit according to the present invention will be described with reference to appended drawings.

Referring to **FIG. 2**(a), when the medical apparatus M moves in the direction A complying with gravity, due to the structure of the links 100, each of the third and fourth joints 116, 118 moves in the direction B and C respectively, therefore, the first and second counterweights 132, 134 independently move in the direction D and E opposite to the direction of gravity. Consequently, the potential energy of the first and second counterweights 132, 134 increases, therefore, the medical apparatus M can move with less force when the medical apparatus M to returns to its original position or moves to other positions as illustrated in **FIG. 2**(b).

Conversely, when the medical apparatus M moves in the direction opposite to gravity as illustrated in **FIG. 2(b)****,** the links 100 function to the opposite direction from **FIG. 2(a)****,** and thus the first and second counterweights 132, 134 are moved in the direction complying with gravity. Therefore, the potential energy is decreased and the medical apparatus M can be moved against gravity with little force.

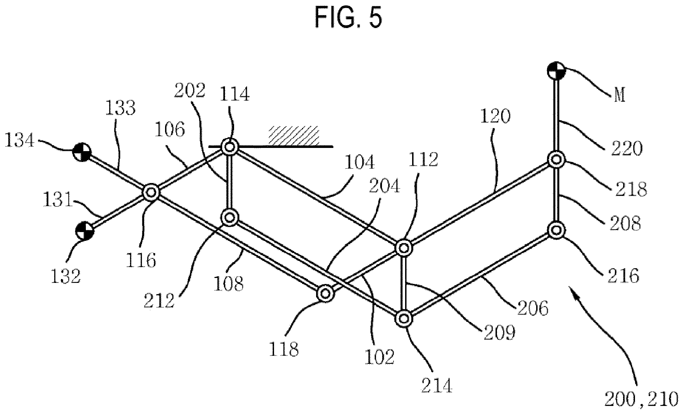

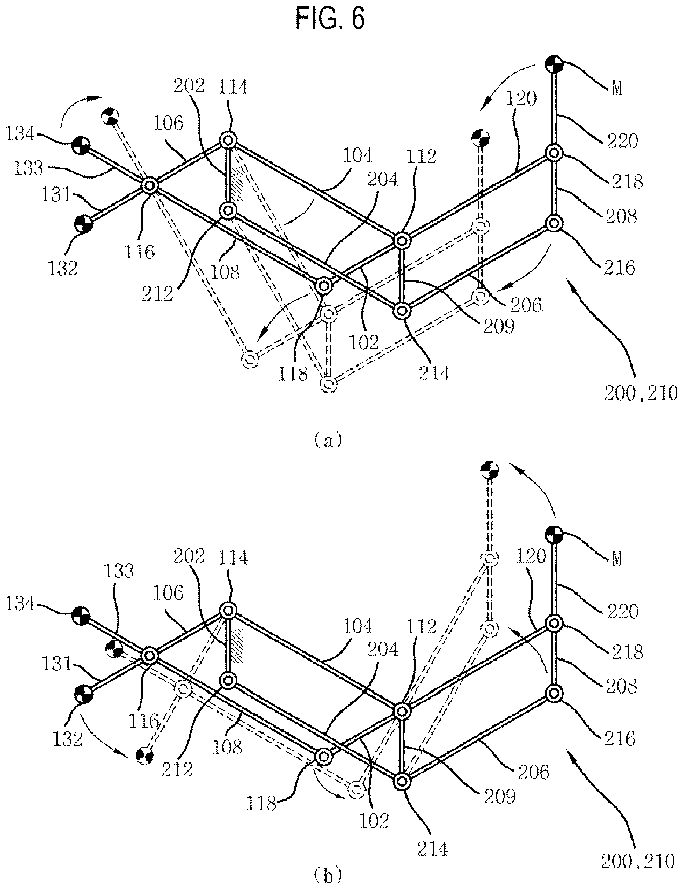

**FIG. 4** is a schematic diagram illustrating a stand equipped with extension link members according to the invention. **FIG. 5** is a schematic diagram illustrating another embodiment of **FIG. 4****.** **FIG. 6** is a schematic diagrams illustrating **FIG. 5** in operation modes.

The extension link members comprise extension links 200 connected to the links 100 and the front link 120; extension joints 210 connected to each connection part of the extension links 200 respectively and capable of mutually rotating the extension links 200; and an extension front link 220 extended from an end of the extension link 200 that is one of extension links 200 and connected to the front link 120, thereby functioning to increase the degree of freedom of the medical apparatus M.

The extension joints 210 comprise first, second, third and fourth extension joints 212, 214,216,218.

The extension links 200 are placed at the opposite side of the second link 104. The extension links 200 comprise a second extension link 204 whose both ends are connected to the first and second extension joints 212, 214; a third extension link 206 placed at the opposite side of the front link 120, one end of the third extension link 206 being connected to the second extension joint 214 and the other end of the third extension link 206 being connected to the third extension joint 216; a first extension link 202 whose both ends are respectively connected to the second joint 114 and the first extension joint 212; a fourth extension link 208 whose one end is connected to the third extension joint 216 and whose the other end is connected to the fourth extension joint 218; the fifth extension link 209 whose both ends are respectively connected to the first joint 112 and the second extension joint 214.

In the extension link members illustrated in **FIG. 4****,** each of the second and third extension links 204, 206 is placed in parallel with the second link 104 and the front link 120 respectively, and one ends of the second and third extension links 204, 206 are connected to each other through the second extension joint 214. Further, the other end of the second extension link 204 is connected to the first extension joint 212 and the other end of the third extension link 206 is connected to the third extension joint 216. In addition, the both ends of the first extension link 202 are connected to the second joint 114 and the first extension joint 212, and the both ends of the fourth extension link 208 are connected to the third extension joint 216 and the fourth extension joint 218. Further, the both ends of the fifth extension link 209 are connected to the first joint 112 and the second extension joint 214. That is, the extension links 200 having the shape of two overlapped parallelograms are extended from the second joint 114 that is the center point.

Also, the first extension link 202 can be fixed perpendicularly to the installation surface in order to maintain an extension front link 220, which will be described below, to be vertical. In other words, since the first extension link 202 and the fifth extension link 209 are always in parallel and the fifth extension link 209 and the fourth extension link 208 are always in parallel, the extension front link 220 extended from the fourth extension link 208 is likewise in parallel with the first extension link 202 all the time. Accordingly, in case where the medical apparatus M such as a microscope should always remain vertical, the medical apparatus M can always remain vertical by vertically fixing the first extension link 202.

The extension front link 220 is extended from the fourth extension link 208, and the medical apparatus M is mounted to the tip of the extension front link 220. After all, the extension links 200 are the supplementary means for connecting the extension front link 220 and the medical apparatus M is interlocked with the movement of the links 100 as well as the movement of the extension link members by the extension front link 220 to increase the degree of freedom.

Meanwhile, as illustrated in **FIG. 5****,** the stand equipped with a counterbalance unit according to the present invention can be formed in the shape in which the top and the bottom of **FIG. 4** are inverted. In this case, the medical apparatus M functions upwardly, and may be used as an objective lens of a microscope. Similarly, as illustrated in **FIGS. 6**(a) and **6**(b), the first extension link 202 can be fixed perpendicularly to the ground and the extension front link 220 can move while always remaining vertical.

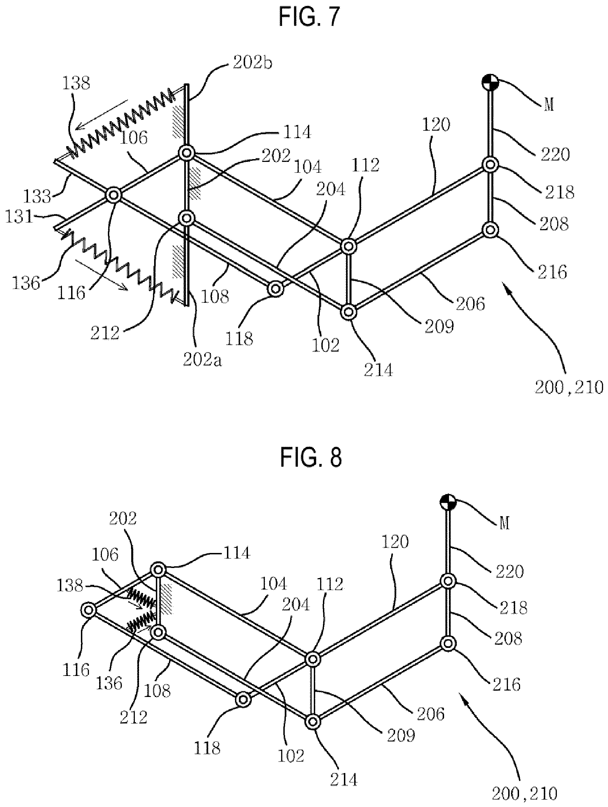

**FIGS. 7** to **9** are schematic diagrams illustrating various embodiments of the counterbalance unit.

Hereinafter, the stand equipped with extension link members, to which counter springs is applied, described with reference to appended drawings.

First, referring to **FIG. 7****,** the stand is provided with the first and second balancing links 131, 133 extended from the third and fourth links 106, 108; and first and the second auxiliary links 202a, 202b extended from the first extension link 202. The first and second auxiliary links 202a, 202b are fixed perpendicularly to the ground like the first extension link 202. And, one end of the first counter spring 136 is connected to the tip of the first balancing link 131, while the other end of the first counter spring 136 is connected to the first auxiliary link 202a. In this case, the first counter spring 136 is a tension spring and moves the first balancing link 131 in the gravitational direction, i.e. the direction of the arrow, to compensate the torque in joints generated by the medical apparatus M. One end of the second counter spring 138 is connected to the tip of the second balancing link 133, while the other end of the second counter spring 138 is connected to the second auxiliary link 202b. In this case, the second counter spring 138 is a compression spring and moves the second balancing link 133 in the direction of the arrow to compensate the torque in joints generated by the medical apparatus M.

Referring to **FIG. 8** as another embodiment, one end of the first counter spring 136 is connected to the fourth link 108, while the other end of the first counter spring 136 is connected to the first extension link 202. One end of the second counter spring 138 is connected to the third link 106, while the other end of the second counter spring 138 is connected to the first extension link 202. The first and second counter springs 136, 138 are tension springs and each of the first and second counter springs 136, 138 moves the fourth link 108 and the third link 106 in the directions of the arrows respectively to compensate the torque in joints generated by the medical apparatus M.

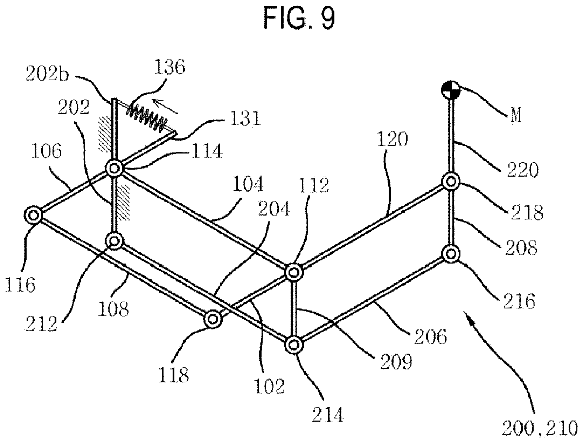

Referring to **FIG. 9** as a further embodiment, the second auxiliary link 202b is extended from the first extension link 202, and the first balancing link 131 is extended from the third link 106 connected to the second joint 114. One end of the first counter spring 136 is connected to the first balancing link 131, while the other end of the first counter spring 136 is connected to the second auxiliary link 202b. In this case, the first counter spring 136 is a tension spring, and moves the first balancing link 131 in the direction of the arrow to compensate the torque in joints generated by the medical apparatus M.

Thus, in case where the counter springs are applied to the stand equipped with extension link members, various embodiments can be made. For the greater variety of embodiments, the stand may be provided with the counter springs that are directly connected to the links 100 or the extension links 200. Further, when necessary, additional balancing links may be added to the stand.

Meanwhile, when necessary, more extension link members can be added to the stand. An additional link is connected to the second joint 114 supported by the holding unit, a link is connected in parallel with the second link 104, and links, each of which is in parallel with the front link 120 and the extension front link 220 respectively, are connected. Next, an additional front link is connected to the extension front link 220 and then the medical apparatus is mounted to the tip of the connected additional front link. In this way, N number of extension link members can be additionally mounted, variously setting the degree of freedom of the medical apparatus.

As the number of extension link members increases, the medical apparatus M gets further from the second joint 114 that is the center point of rotation, accordingly, the torque in joints generated by the medical apparatus M grows bigger. In order to maintain a stable counterbalance, it is desirable that the lengths of the first and second balancing links 131, 133 increase in proportion to the growing number of the extension link members. For the efficient counter balance, it is more preferable to increase the weight of the first and second counterweights 132, 134 as well as the lengths of the first and second balancing links 131, 133.

**[Description of reference symbols]**

| | |
|---|---|
| 100: link | 102: first link |
| 104: second link | 106: third link |
| 108: fourth link | 110: joint |
| 112: first joint | 114: second joint |
| 116: third joint | 118: fourth joint |
| 120: front link | 130: counterbalance unit |
| 131: first balancing link | 132: first counterweight |
| 133: second balancing link | 134: second counterweight |
| 136: first counter spring | 138: second counter spring |
| 200: extension link | 202: first extension link |
| 202a: first auxiliary link | 202b: second auxiliary link |
| 204: second extension link | 206: third extension link |
| 208: fourth extension link | 209: fifth extension link |
| 210: extension joint | 212: first extension joint |
| 214: second extension joint | 216: third extension joint |
| 218: fourth extension joint | 220: extension front link |

## Claims

1. A stand equipped with a counterbalance unit, comprising:
first to fourth links (102, 104, 106, 108) arranged in a parallelogram configuration;
first to fourth joints (112, 114, 116, 118), each of the first to fourth joints (112, 114, 116, 118) being connected to each connection part of the first to fourth links (102, 104, 106, 108) respectively and enabling the first to fourth links (102, 104, 106, 108) to be mutually rotatable, wherein the second joint (114) between the second link (104) and the third link (106) is fixed to a holding unit and the second joint (114) and the fourth joint (118) are placed in a diagonal direction;
a front link (120) extended from the first link (102) in a direction from the fourth joint (118) toward the first joint (112), the tip of the front link being configured for mounting a medical apparatus thereon;
a counterbalance unit (130) connected to the third joint (116), the stand further comprising:
a first extension link (202) connected to the second joint (114);
a first extension joint (212) placed at an end of the first extension link (202);
a second extension link (204) extended from the first extension joint (212) in parallel with the second link (104);
a second extension joint (214) placed at an end of the second extension link (204);
a third extension link (206) extended from the second extension joint (214) in parallel with the front link (120);
a third extension joint (216) placed at an end of the third extension link (206);
a fourth extension joint (218) placed at an end of the front link (120);
a fourth extension link (208) placed between the third extension joint (216) and the fourth extension joint (218); and
a fifth extension link (209) placed between the first joint (112) and the second extension joint (214),
wherein the first extension link (202), the fourth extension link (208) and the fifth extension link (209) are in parallel with one another.

2. The stand equipped with a counterbalance unit of claim 1, further comprising
an extension front link (220) extended from the fourth extension link (208).

3. The stand equipped with a counterbalance unit of claim 1, wherein the counterbalance unit (130) comprises first and second balancing links (131, 133) and first and second counterweights (132, 134) mounted to tips of the first and second balancing links (131, 133) respectively.

4. The stand equipped with a counterbalance unit of claim 3, wherein the first and second balancing links (131, 133) are connected to the third joint (116), and each of the first and second balancing links (131, 133) is extended from the third and fourth links (106, 108) respectively.

5. The stand equipped with a counterbalance unit of claim 1, wherein the counterbalance unit (130) comprises first and second balancing links (131, 133) and first and second counter springs (136, 138),
wherein the first counter spring (136) is placed between the first balancing link (131) and the holding unit, and
wherein the second counter spring (138) is placed between the second balancing link (133) and the holding unit.

6. The stand equipped with a counterbalance unit of claim 1, wherein the first extension link (202) is fixed to the holding unit.

7. The stand equipped with a counterbalance unit of claim 1,
wherein the counterbalance unit (130) comprises:
a first counter spring (136) placed between the fourth link (108) and the holding unit; and a second counter spring (138) placed between the third link (106) and the holding unit.

## Patentansprüche

1. Stativ ausgestattet mit einer Ausgleichsgewichtseinheit, umfassend:
erste bis vierte Glieder (102, 104, 106, 108), die in einer Parallelogramm-Konfiguration angeordnet sind;
erste bis vierte Gelenke (112, 114, 116, 118), wobei jedes der ersten bis vierten Gelenke (112, 114, 116, 118) jeweils mit jedem Verbindungsteil der ersten bis vierten Glieder (102, 104, 106, 108) verbunden ist und ermöglicht, dass die ersten bis vierten Glieder (102, 104, 106, 108) gegeneinander drehbar sind, wobei das zweite Gelenk (114) zwischen dem zweiten Glied (104) und dem dritten Glied (106) an einer Halteinheit befestigt ist und das zweite Gelenk (114) und das vierte Gelenk (118) in einer diagonalen Richtung angeordnet sind;
ein vorderes Glied (120), das sich von dem ersten Glied (102) in einer Richtung von dem vierten Gelenk (118) zu dem ersten Gelenk (112) erstreckt, wobei die Spitze des vorderen Glieds konfiguriert ist, um ein medizinisches Gerät darauf aufzubringen;
eine Ausgleichsgewichtseinheit (130), die mit dem dritten Gelenk (116) verbunden ist,
wobei das Stativ ferner umfasst:
ein erstes Verlängerungsglied (202), das mit dem zweiten Gelenk (114) verbunden ist;
ein erstes Verlängerungsgelenk (212), das an einem Ende des ersten Verlängerungsglieds (202) angeordnet ist;
ein zweites Verlängerungsglied (204), das sich von dem ersten Verlängerungsgelenk (212) parallel zu dem zweiten Glied (104) erstreckt;
ein zweites Verlängerungsgelenk (214), das an einem Ende des zweiten Verlängerungsglieds (204) angeordnet ist;
ein drittes Verlängerungsglied (206), das sich von dem zweiten Verlängerungsgelenk (214) parallel zu dem vorderen Glied (120) erstreckt;
ein drittes Verlängerungsgelenk (216), das an einem Ende des dritten Verlängerungsglieds (206) angeordnet ist;
ein viertes Verlängerungsgelenk (218), das an einem Ende des vorderen Glieds (120) angeordnet ist;
ein viertes Verlängerungsglied (208), das zwischen dem dritten Verlängerungsgelenk (216) und dem vierten Verlängerungsgelenk (218) angeordnet ist; und
ein fünftes Verlängerungsglied (209), das zwischen dem ersten Gelenk (112) und dem zweiten Verlängerungsgelenk (214) angeordnet ist,
wobei das erste Verlängerungsglied (202), das vierte Verlängerungsglied (208) und das fünfte Verlängerungsglied (209) parallel zueinander sind.

2. Das Stativ ausgestattet mit einer Ausgleichsgewichtseinheit nach Anspruch 1, ferner umfassend ein vorderes Verlängerungsglied (220), das sich von dem vierten Verlängerungsglied (208) erstreckt.

3. Das Stativ ausgestattet mit einer Ausgleichsgewichtseinheit nach Anspruch 1, wobei die Ausgleichsgewichtseinheit (130) erste und zweite Ausgleichsglieder (131, 133) und erste und zweite Ausgleichsgewichte (132, 134) umfasst, die jeweils an den Spitzen der ersten und zweiten Ausgleichsglieder (131, 133) angebracht sind.

4. Das Stativ ausgestattet mit einer Ausgleichsgewichtseinheit nach Anspruch 3, wobei die ersten und zweiten Ausgleichsglieder (131, 133) mit dem dritten Gelenk (116) verbunden sind, und jedes der ersten und zweiten Ausgleichsglieder (131, 133) sich jeweils von den dritten und vierten Gliedern (106, 108) erstreckt.

5. Das Stativ ausgestattet mit einer Ausgleichsgewichtseinheit nach Anspruch 1, wobei die Ausgleichsgewichtseinheit (130) erste und zweite Ausgleichsglieder (131, 133) und erste und zweite Gegenfedern (136, 138) umfasst,
wobei die erste Gegenfeder (136) zwischen dem ersten Ausgleichsglied (131) und der Halteeinheit angeordnet ist, und
wobei die zweite Gegenfeder (138) zwischen dem zweiten Ausgleichsglied (133) und der Halteinheit angeordnet ist.

6. Das Stativ ausgestattet mit einer Ausgleichsgewichtseinheit nach Anspruch 1, wobei das erste Verlängerungsglied (202) an der Halteeinheit befestigt ist.

7. Das Stativ ausgestattet mit einer Ausgleichsgewichtseinheit nach Anspruch 1, wobei die Ausgleichsgewichtseinheit (130) umfasst:
eine erste Gegenfeder (136), die zwischen dem vierten Glied (108) und der Halteeinheit angeordnet ist; und eine zweite Gegenfeder (138), die zwischen dem dritten Glied (106) und der Halteieinheit angeordnet ist.

## Revendications

1. Support équipé d'une unité d'équilibrage, comprenant :
des première à quatrième liaisons (102, 104, 106, 108) agencées selon une configuration en parallélogramme,
des première à quatrième articulations (112, 114, 116, 118), chacune des première à quatrième articulations (112, 114, 116, 118) étant reliée à chaque pièce de connexion des première à quatrième liaisons (102, 104, 106, 108) respectivement et permettant aux première à quatrième liaisons (102, 104, 106, 108) de pouvoir être en rotation mutuellement, dans lequel la deuxième articulation (114) entre la deuxième liaison (104) et la troisième liaison (106) est fixée à une unité de retenue et la deuxième articulation (114) et la quatrième articulation (118) sont placées dans une direction en diagonale,
une liaison frontale (120) qui s'étend à partir de la première liaison (102) dans une direction entre la quatrième articulation (118) et la première articulation (112), l'extrémité de la liaison frontale étant configurée pour monter un appareil médical sur celle-ci,
une unité d'équilibrage (130) reliée à la troisième articulation (116), le support comprenant en outre :
une première liaison d'extension (202) reliée à la deuxième articulation (114),
une première articulation d'extension (212) placée au niveau d'une extrémité de la première liaison d'extension (202),
une deuxième liaison d'extension (204) qui s'étend à partir de la première articulation d'extension (212) parallèlement à la deuxième liaison (104),
une deuxième articulation d'extension (214) placée au niveau d'une extrémité de la deuxième liaison d'extension (204),
une troisième liaison d'extension (206) qui s'étend à partir de la deuxième articulation d'extension (214) parallèlement à la liaison frontale (120),
une troisième articulation d'extension (216) placée au niveau d'une extrémité de la troisième liaison d'extension (206),
une quatrième liaison d'extension (218) placée au niveau d'une extrémité de la liaison frontale (120),
une quatrième liaison d'extension (208) placée entre la troisième articulation d'extension (216) et la quatrième articulation d'extension (218), et
une cinquième liaison d'extension (209) placée entre la première articulation (112) et la deuxième articulation d'extension (214),
dans lequel la première liaison d'extension (202), la quatrième liaison d'extension (208) et la cinquième liaison d'extension (209) sont parallèles les unes aux autres.

2. Support équipé d'une unité d'équilibrage selon la revendication 1, comprenant en outre :
une liaison d'extension frontale (220) qui s'étend à partir de la quatrième liaison d'extension (208).

3. Support équipé d'une unité d'équilibrage selon la revendication 1, **caractérisé en ce que** l'unité d'équilibrage (130) comprend des première et seconde liaisons d'équilibrage (131, 133) et des premier et second contrepoids (132, 134) montés sur les extrémités des première et seconde liaisons d'équilibrage (131, 132) respectivement.

4. Support équipé d'une unité d'équilibrage selon la revendication 3, **caractérisé en ce que** les première et seconde liaisons d'équilibrage (131, 133) sont reliées à la troisième articulation (116), et chacune des première et seconde liaisons d'équilibrage (131, 133) s'étend à partir des troisième et quatrième liaisons (106, 108) respectivement.

5. Support équipé d'une unité d'équilibrage selon la revendication 1, **caractérisé en ce que** l'unité d'équilibrage (130) comprend des première et seconde liaisons d'équilibrage (131, 133) et des premier et second contre-ressorts (136, 138),
**en ce que** le premier contre-ressort (136) est placé entre la première liaison d'équilibrage (131) et l'unité de retenue, et
**en ce que** le second contre-ressort (138) est placé entre la seconde liaison d'équilibrage (133) et l'unité de retenue.

6. Support équipé d'une unité d'équilibrage selon la revendication 1, **caractérisé en ce que** la première liaison d'extension (202) est fixée à l'unité de retenue.

7. Support équipé d'une unité d'équilibrage selon la revendication 1,
**caractérisé en ce que** l'unité d'équilibrage (130) comprend :
un premier contre-ressort (136) placé entre la quatrième liaison (108) et l'unité de retenue, et
un second contre-ressort (138) placé entre la troisième liaison (106) et l'unité de retenue.
